**Europäisches Patentamt**

**European Patent Office**

**Office européen des brevets**

(11) Veröffentlichungsnummer: **0 047 462**
**A2**

(12) # EUROPÄISCHE PATENTANMELDUNG

(21) Anmeldenummer: 81106790.9

(22) Anmeldetag: 31.08.81

(51) Int. Cl.³: **A 61 L 2/20**
// A61K35/16

(30) Priorität: 10.09.80 DE 3033932

(43) Veröffentlichungstag der Anmeldung: 17.03.82
Patentblatt 82/11

(84) Benannte Vertragsstaaten: **AT BE CH DE FR GB IT LI NL SE**

(71) Anmelder: **Biotest-Serum-Institut GmbH,
Flughafenstrasse 4, D-6000 Frankfurt-Niederrad (DE)**

(72) Erfinder: **Kotitschke, Ronald, Dr.,Dipl.-Chem.,
Kleiststrasse 23, D-6072 Dreieich (DE)**
Erfinder: **Wolfgang, Stephan, Dr.,Dipl.-Chem.,
Philipp-Holzmannstrasse 84, D-6072 Dreieich (DE)**

(74) Vertreter: **Beil, Walter, Dr. et al, BEIL, WOLFF & BEIL
Rechtsanwälte Adelonstrasse 58, D-6230 Frankfurt am
Main 80 (DE)**

(54) Verfahren zur Kaltsterilisation von Blutgerinnungsfaktor VIII enthaltenden Präparaten.

(57) Die Erfindung betrifft ein Verfahren zur Kaltsterilisation von Blutgerinnungsfaktor VIII enthaltenden pharmazeutischen Präparaten unter Behandlung mit $\beta$-Propiolacton und Bestrahlung mit UV-Licht, das dadurch gekennzeichnet ist, daß man eine Faktor VIII enthaltende Proteinlösung (A) mit einem physiologisch verträglichen, nichtionogenen Tensid behandelt; (B) einer Bestrahlung mit UV-Licht unterwirft; (C) mit $\beta$-Propiolacton behandelt und (D) einer Adsorptionsbehandlung mit kolloidaler Kieselsäure unterwirft, wobei die Stufen B–C–D auch in der Reihenfolge B–D–C oder D–C–B oder D–B–C durchgeführt werden können. Die nach dem erfindungsgemäßen Verfahren erhaltenen Präparate sind hepatitis-sicher.

0047462

Unsere Nr. 23 430                                    Ec/br


Biotest-Serum-Institut GmbH
Flughafenstraße 4
6000 Frankfurt-Niederrad

Verfahren zur Kaltsterilisation von Blutgerinnungsfaktor VIII enthaltenden Präparaten

Die Erfindung betrifft das in den Patentansprüchen
gekennzeichnete Verfahren zur Kaltsterilisation von
Blutgerinnungsfaktor VIII enthaltenden Präparaten.

Zur Therapie angeborener oder erworbener Gerinnungsstörungen, die auf einen Mangel oder Defekt plasmatischer Gerinnungsfaktoren zurückzuführen sind, eignen
sich Präparate, die diese Faktoren in ankonzentrierter
Form enthalten. Zu diesen Konzentraten gehören Fraktionen, die aus Blutplasma hergestellt werden. Die zur

- 2 -

Zeit gebräuchlichsten Fraktionen sind eine durch Alkohol-Fraktionierung nach Cohn (J.Am.Chem.Soc., Bd. 72 (1950), S.465) erhaltene Cohn-Fraktion I, ein durch Kälteausfällung erhaltenes Kryopräzipitat, eine Fraktion, die den Faktor VIII (= antihaemophiles Globulin A = AHG) enthält, und ein AHG-Konzentrat, enthaltend antihaemophiles Globulin A und den Prothrombinkomplex, d.h. die Faktoren II, VII, IX und X (PPSB). Bei all diesen Präparaten besteht das Risiko, prinzipiell Hepatitis zu übertragen.

Der Radioimmuntest auf Hepatitis-Oberflächen-Antigen (HBsAg) ist um das 100- bis 1000-fache zu unempfindlich, so daß auch HBsAg-negative Blutprodukte, insbesondere AHG- und PPSB-Präparate, zu einem hohen Prozentsatz infektiös sind und die Hepatitis B übertragen können (vgl. J.H. Hoofnagle u.a., "The Prevalence of Hepatitis B Surface Antigen in Commercially Prepared Plasma Products", J.Lab.Clin. Med., Bd. 88 (1976), S. 102 bis 112 und R.J.Wyke u.a. "Transmission of Non A - Non B Hepatitis to Chimpanzees by Factor IX Concentrates after Fatal Complications in Patients with Chronic Liver Disease", The Lancet 1 (1979), Nr. 8115, S. 520 bis 524).

Da es durch diagnostische Maßnahmen nicht gelingt, hepatitissichere Blutprodukte aus Plasmapools zu erzielen, kommt der Sterilisation von Blut und Blutbestandteilen zentrale Bedeutung zu. Humanalbumin wird z.B. durch

0047462

- 3 -

Pasteurisierung, d.h. Erhitzen für 10 Stunden auf 60°C, hepatitissicher. Gerinnungsfaktoren können jedoch nicht pasteurisiert werden, da sie schon durch einstündiges Erhitzen auf 60°C zerstört werden. Daher mußte eine Sterilisationsmethode entwickelt werden, die nicht bei hohen, sondern bei niedrigen Temperaturen arbeitet. Eine solche Methode wurde von LoGrippo vorgeschlagen (vgl. G.A. LoGrippo u.a., "Chemical Sterilization of Whole Blood and Plasma with Beta-Propiolactone", Hepatitis Frontiers, Henry Ford Hospital, Int. Symposium 1956, Little,Brown and Comp., Boston, Mass. (1957), S. 371 bis 385 und G.A. LoGrippo u.a. "Chemical and Combined Methods for Plasma Sterilization", Bibl. Haematol., Bd. 7.(1958), S. 225 bis 230). Diese Methode bestand in der Behandlung von Humanplasma mit ß-Propiolacton, die mit einer Bestrahlung mit UV-Strahlen kombiniert werden konnte. Sie wurde auch als "Kaltsterilisation" bezeichnet, weil sie bei Temperaturen unter 37°C durchgeführt wurde.

Die Anwendung der von LoGrippo angegebenen Sterilisationsbedingungen führt jedoch zu hohen Verlusten der Faktor-VIII-Aktivität im Plasma, wie in der folgenden Tabelle I gezeigt wird.

0047462

- 4 -

Tabelle I

Einfluß der Bedingungen der Sterilisation
nach Lo Grippo auf die Faktor-VIII-Aktivität
im Plasma.

| | Faktor VIII in % d.N. |
|---|---|
| frisches Citratplasma | 100 |
| nach Behandlung mit ß-Propio-lacton (0,3 %) | 55 |
| Nach Bestrahlung mit UV (2m Watt/cm² x Min.) | 30 |

Aus ß-Propiolacton/UV-behandeltem Plasma läßt sich Kryopräzipitat nur noch in unzureichenden Ausbeuten gewinnen. Die ß-Propiolacton/UV-Behandlung von Extrakten von Kryopräzipitat ist möglich, führt jedoch unter Anwendung der von LoGrippo angegebenen Bedingungen zu großen Verlusten der Faktor-VIII-Aktivität.

Von J.T. Sgouris u.a. "Stability of Factor I (Fibrinogen) and Factor VIII (Antihemophilic Globulin) to Beta-Propiolactone", Vox Sang., Bd. 8 (1963), S.105 bis 108; W. Doleschel u.a. "The Influence of Beta-Propiolactone on the Coagulability of Human Fibrinogen", Pharmacology, Bd. 2 (1969), S. 1 bis 8; und H. Brunner u.a. "Experimentelle Fibrinogenopathie nach Reaktion von humanem Fibrinogen mit ß-Propiolacton:

Antithrombinaktivität, Wirkung auf die Plättchenaggregation und Funktion auf die Fibrinverfestigungsphase", Verhandlungen der Deutschen Gesellschaft für Innere Medizin, Bd. 79 (1973), S. 1130 bis 1134, wurde versucht, Gerinnunsfaktoren, insbesondere Faktor VIII und Fibrinogen enthaltende Proteinfraktionen, mit ß-Propiolacton zu sterilisieren.

Diese Arbeiten kamen jedoch zu dem Ergebnis, daß die von LoGrippo beschriebene Methode der Sterilisation von Plasma oder Serum mit ß-Propiolacton und UV-Bestrahlung nicht für die Herstellung von Faktor VIII enthaltenden Präparaten oder Fibrinogen geeignet ist, da diese Proteine entweder zu große Aktivitätsverluste erleiden oder aber die von LoGrippo angegebene ß-Propiolactonkonzentration so weit verringert werden muß, daß eine ausreichende viruzide Wirkung nicht erzielt wird.

Bei der Vaccineherstellung ist es üblich, zur Virusinaktivierung verschiedene Inaktivierungsmaßnahmen miteinander zu kombinieren, um auf diese Weise "resistente Fraktionen" zu beseitigen, die gegen eine einzige Inaktivierungsbehandlung beständig sein könnten, die jedoch durch eine zweite oder dritte Behandlung, deren Inaktivierungsmechanismen von demjenigen der ersten Behandlung verschieden ist, inaktiviert wird.

Der Erfindung liegt die Aufgabe zugrunde, Präparate, die den Blutgerinnungsfaktor VIII enthalten, z.B. eine durch Extraktion von Kryopräzipitat erhaltene Fraktion mit Gehalt an Faktor VIII oder eine Cohn-Fraktion I, die als infektiös angesehen werden müssen, mit Hilfe mehrerer chemischer und/oder physikalischer Virusinaktivierungsmaßnahmen und/oder Virusadsorptionsmaßnahmen derart kalt zu sterilisieren, daß einerseits das Infizierungsvermögen inaktiviert wird, daß aber andererseits die biologische Wirksamkeit des Faktor-VIII-Proteins möglichst wenig beeinträchtigt wird.

Diese Aufgabe wird erfindungsgemäß dadurch gelöst, daß man eine Faktor VIII enthaltende Proteinlösung

A mit einem physiologisch verträglichen, nicht ionogenen Tensid behandelt;

B einer Bestrahlung mit UV-Licht unterwirft;

C mit ß-Propiolacton behandelt und.

D einer Adsorptionsbehandlung mit kolloidaler Kieselsäure unterwirft,

wobei die Stufen B-C-D auch in der Reihenfolge B-D-C oder D-C-B oder D-B-C durchgeführt werden können.

Der Begriff der Sterilität ist nicht klar definiert. Es ist deshalb immer die Methode anzugeben, nach der die Sterilität erzielt wurde. Das erfindungsgemäße Verfahren ist eine Methode zur Erzielung von Sterilität.

Im erfindungsgemäßen Verfahren kann als Ausgangsmaterial eine Faktor VIII-haltige Proteinlösung verwendet werden, die durch Isolierung von Kryopräzipitat (Kälteausfällung) aus einem mit einem Blutstabilisator stabilisierten Blutplasma nach bekannten Verfahren (vgl. J.G. Pool u.a., Federation Proc., Bd. 24 (1965), S. 512, Abstr.2122) und Aufarbeitung des Kryopräzipitates nach bekannten Methoden (vgl. z.B. A.J. Johnson u.a. "Preparation of and Clinical Experience with Antihemophilic Factor Concentrates", Thrombos. Diathes. haemorrh., Suppl. 35 (1969), S. 49 bis 59; J. Newman u.a. "Methods for the Production of Clinically Effective Intermediate- and High-Purity Factor-VIII Concentrates", Brit. J. of Haemathology, Bd. 21 (1971), S. 1 bis 20; und R.H. Wagner u.a. "Purification of Antihemophilic Factor (Factor VIII) by Amino Acid Precipitation", Thrombos. Diathes. haemorrh., Bd. 11 (1964), S.64 bis 74) erhalten wurde. Es kann auch eine Cohn-Fraktion I verwendet werden.

Die auf diese Weise erhaltenen Faktor-VIII-haltigen Lösungen enthalten häufig neben angereichertem Faktor VIII auch Fibrinogen in stark angereicherter Form.

Die erfindungsgemäße Behandlung der Virusinaktivierung bzw. -elimination besteht in der Kombination von vier aufeinanderfolgenden Maßnahmen, von denen jede für sich die Faktor-VIII-Aktivität nicht wesentlich beeinträchtigt.

Die Wirkung der einzelnen Maßnahmen auf die Faktor-VIII- Aktivität wird nachfolgend näher erläutert.

## Tensid-Behandlung

Eine nach Extraktion von Kryopräzipitat erhaltene Faktor-VIII-Fraktion oder eine andere Faktor VIII enthaltende Proteinlösung wird mit einem physiologisch verträglichen, nichtionogenen Tensid in einer Konzentration von 1,0 bis 5,0 % versetzt und bei Zimmertemperatur behandelt. Als Tenside können z.B. Sorbitanfettsäureester und deren Polyoxyethylenderivate verwendet werden.

Vorzugsweise verwendet man Sorbitanmono- und -trifettsäureester basierend auf Laurin-, Palmitin-, Stearin- und Ölsäure sowie die entsprechenden Polyoxyethylenderivate (Handelsnamen für diese Produkte sind Span$^R$ und TWEEN$^R$). Die gute Wasserlöslichkeit der Polyoxyethylenderivate und die gute Verträglichkeit dieser Sorbitan-fettsäureester allgemein ($LD_{50} > 37$ g/kg bei Ratten) begünstigen die bevorzugte Verwendung dieser Tenside im erfindungsgemäßen Verfahren.

Ein Einfluß dieser Tenside auf die Faktor-VIII-Aktivität ist bei Verwendung von Konzentrationen bis zu einer Gesamtkonzentration von 3 % in der zu behandelnden Lösung nicht nachweisbar, wie in Tabelle II gezeigt wird.

0047462

- 9 -

## Tabelle II

Abhängigkeit der Faktor-VIII-Aktivität von der Tensid-Konzentration

| | Tensid-Konzentration, % | | |
| --- | --- | --- | --- |
| | Sorbitan-oleat (Span[R] 85) | Polyoxyethylen-20-sorbitan-monooleat (TWEEN[R] 80) | Faktor-VIII (% d.N.) |
| Ausgangsgemisch | 0 | 0 | 500 |
| 1 Std., Raumtemperatur | 1 | 1 | 500 |
| 1 Std., Raumtemperatur | 2 | 2 | 500 |
| 1 Std., Raumtemperatur | 3 | 3 | 450 |

## Adsorptionsbehandlung mit kolloidaler Kieselsäure

Bei dem Versuch, kolloidale Kieselsäure zur Adsorption möglicher vorhandener Viren in der Faktor-VIII-Lösung zu verwenden, wurde überraschenderweise gefunden, daß die Faktor-VIII-Aktivität sowie das Fibrinogen nach der Adsorption weitgehend in der Faktor-VIII-Lösung erhalten bleiben.

Daß Viren an kolloidale Kieselsäure gebunden werden können, ist bekannt (DE-PS 1 617 319). Bekannt ist aber auch, daß kolloidale Kieselsäure zur Adsorption von Fibrinogen und instabilen Proteinen verwendet wird (DE-OS 29 02 158).

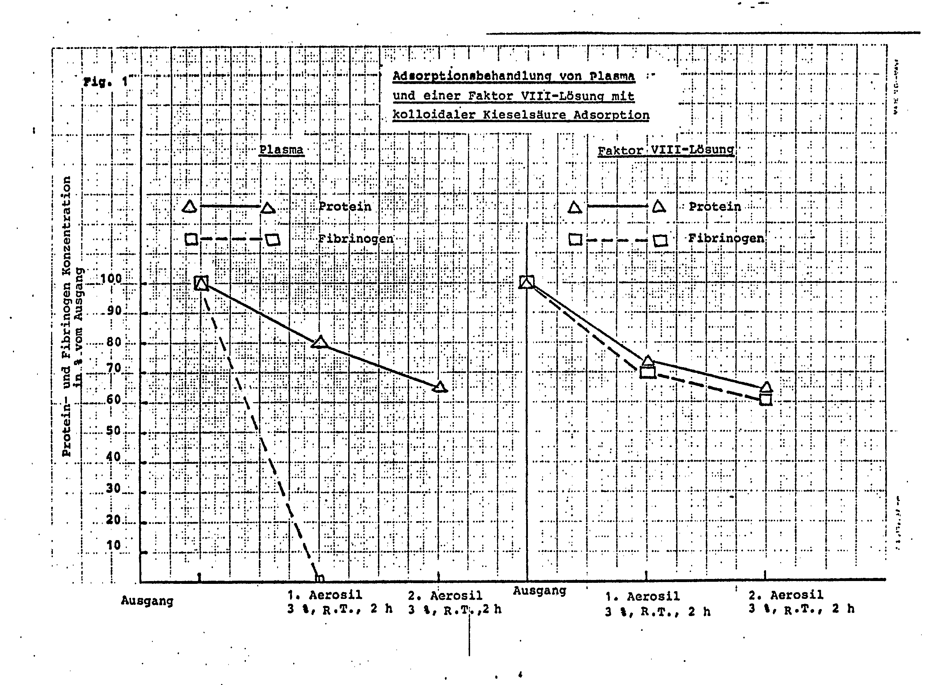

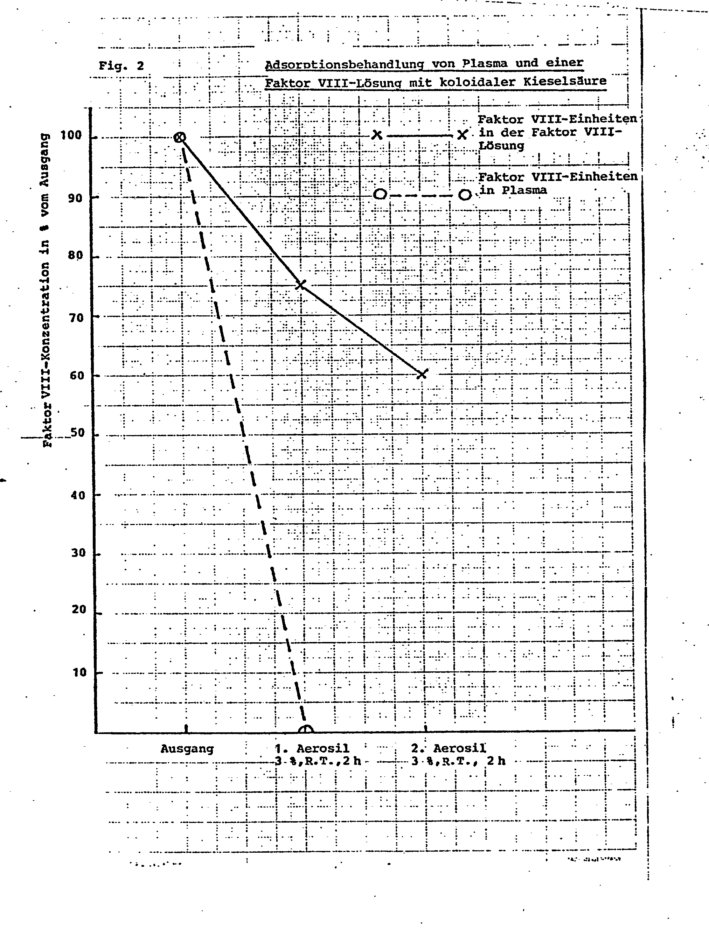

Bei der Adsorptionsbehandlung einer Faktor-VIII-Konzentrat-Lösung mit kolloidaler Kieselsäure wurde überraschenderweise weder Fibrinogen noch der Faktor VIII aus der Faktor-VIII-Konzentrat-Lösung an die Kieselsäure adsorbiert. Dieser Befund steht im krassen Gegensatz zu den bekannten Ergebnissen, nach denen Faktor VIII und Fibrinogen durch kolloidale Kieselsäure vollständig adsorbiert werden. Die Ergebnisse entsprechender Versuche sind in den Fig. 1 und 2 dargestellt. Die in Fig. 2 auftretenden Verluste erklären sich durch den Flüssigkeitsverlust während der Adsorption. Die nachstehende Tabelle III zeigt, daß die Aktivität von Faktor VIII und die Fibrinogen-Konzentration der Faktor-VIII-haltigen Lösung vor und nach der Adsorption mit kolloidaler Kieselsäure nahezu unverändert sind.

## Tabelle III

Abhängigkeit der Faktor-VIII-Aktivität und Fibrinogen-Konzentration von der Adsorption mit kolloidaler Kieselsäure.

|  | Volumen (ml) | Faktor VIII (% d.N.) | Fibrinogen (mg %) |
| --- | --- | --- | --- |
| Ausgangsgemisch | 500 | 3000 | 1950 |
| nach Adsorption mit kolloidaler Kieselsäure (3 %, 2 Std., Raumtemperatur) | 360 | 3000 | 1850 |

Die für die Adsorptionsbehandlung verwendete kolloidale Kieselsäure sollte vorzugsweise eine spezifische Oberfläche von 50 bis 400 m²/g aufweisen und in einer Konzentration von 1 bis 3 g pro g Protein eingesetzt werden. Als kolloidale Kieselsäure kann beispielsweise ein unter dem Warenzeichen "Aerosil" vertriebenes Handelsprodukt verwendet werden.

Die Adsorption mit kolloidaler Kieselsäure kann wiederholt werden und die Adsorptionstemperatur sogar auf 45°C erhöht werden, ohne die bei Raumtemperatur erzielten Ergebnisse wesentlich zu verändern.

UV-Bestrahlung

Die UV-Bestrahlung der Faktor-VIII-Lösung führt zu einem Aktivitätsverlust von etwa 20 %. Im Vergleich dazu beträgt der Aktivitätsverlust des Faktor VIII im Verband der Plasmaproteine nach vorhergehender ß-Propiolactonbehandlung bei Anwendung der von LoGrippo vorgeschlagenen Konzentrationen nahezu 50 %, wie bereits in Tabelle I gezeigt wurde.

Behandlung mit ß-Propiolacton und UV-Bestrahlung

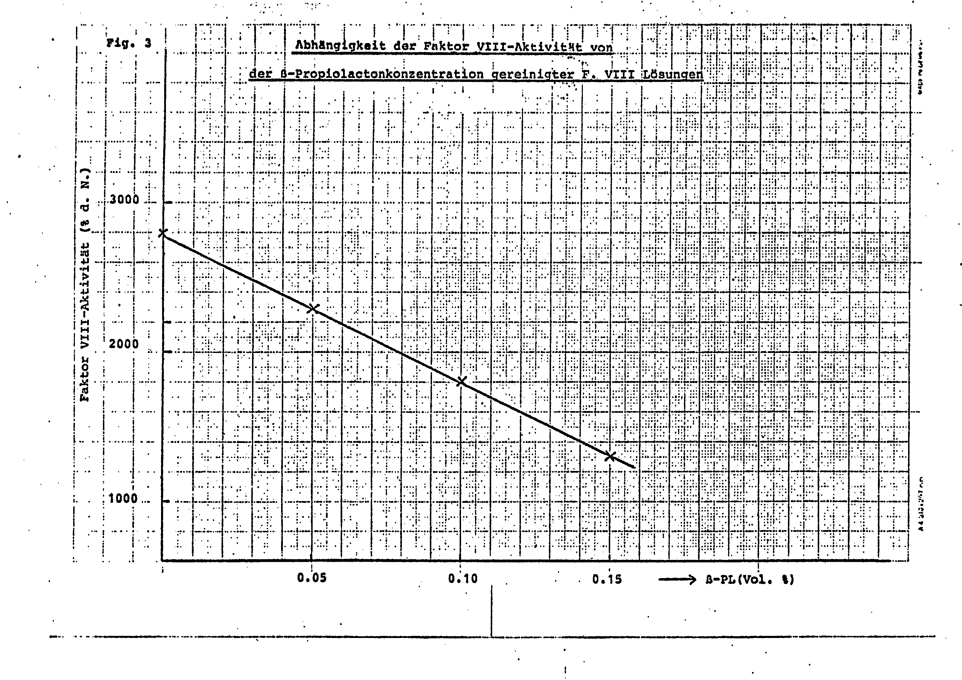

Ein Erhalt der Faktor-VIII-Aktivität von Faktor-VIII-haltigen Lösungen ist nur durch eine Änderung der von LoGrippo angegebenen Bedingungen zu erreichen. Die Abhängigkeit der Faktor-VIII-Aktivität von verschiedenen ß-Propiolactonkonzentrationen ist in Fig. 3 dargestellt. Bei einer ß-Propiolacton-Konzentration von 0,05 % beträgt der Aktivitätsverlust einer Faktor-VIII-haltigen

- 12 -

Lösung mit Proteinkonzentrationen von 2 bis 5 g/100 ml etwa 20 %. Im erfindungsgemäßen Verfahren sollte die Konzentration des ß-Propiolactons 0,012 bis 0,067 g pro g Protein betragen.

Die anschließende UV-Bestrahlung (Dosis nach LoGrippo; d.h. 2 mWatt/cm² x Min.) verursacht noch einmal einen Aktivitätsverlust von etwa 20 %, wie der folgenden Tabelle IV zu entnehmen ist.

Tabelle IV

Einfluß der ß-Propiolacton/UV-Behandlung auf die Faktor-VIII-Aktivität von Faktor-VIII-haltigen Lösungen.

|  | Faktor VIII, % d.N. | Verbleibende Faktor-VIII-Aktivität, % |
|---|---|---|
| Ausgangslösung | 2 800 | 100 |
| ß-Propiolacton (0,05 %) | 2 300 | 82 |
| UV (2 mWatt/cm² x Min.) | 1 900 | 67 |

Um das gesamte erfindungsgemäße Kaltsterilisationsverfahren in Bezug auf den Verbleib der Hepatitisviren zu prüfen, wurde einem Kryopräzipitat-Extrakt ein HBsAgpositives Plasma zugegeben und die HBs Ag-Konzentration anschließend in den verschiedenen Fraktionen bestimmt.

Tabelle V zeigt die Ergebnisse für eine Durchführungsform mit der Stufenfolge A-B-C-D.

Tabelle V

Quantitative HBs Ag-Verteilung bei der Aufarbeitung von Kryopräzipitat, dem HBs Ag zugesetzt worden war.

| Virusinaktivierungsmaßnahmen | HBs Ag (ng/ml) |
|---|---|
| Ausgangsgemisch (Kryopräzipitat) | 2 700 |
| 1. nach Tensid-Behandlung | 170 |
| 2. nach UV-Bestrahlung | 90 |
| 3. nach ß-Propiolacton-Behandlung | 75 |
| 4. nach Adsorptionsbehandlung mit kolloidaler Kieselsäure | Ø |

Da in-vitro-Versuche mit dem Hepatitisvirus bei heutigem Stand der Technik nicht durchführbar sind, wurde als geeignetes Modell für das Hepatitisvirus der von LoGrippo vorgeschlagene Coli-Phage (G.A. LoGrippo: Investigation. Annals of the New York Acad. of Sciences, Bd. 83 (1960), Art. 4, S. 578 bis 595) zur Prüfung der Wirksamkeit des Verfahrens verwendet.

Dazu wurden einem Kryopräzipitat-Extrakt so viel coli $T_2$-Phagen zugesetzt, bis die Ausgangskonzentration an Viren $10^5$/ml war.

Tabelle VI zeigt die Ergebnisse für eine Durchführungsform mit der Stufenfolge A-D-C-B.

0047462

- 14 -

## Tabelle VI

### Phagenverteilung bei der Aufarbeitung von Kryopräzipitat, dem Phagen zugesetzt worden waren.

| Virusinaktivierungsmaßnahmen | Phagenkonzentration (Viren/ml) |
|---|---|
| Ausgangsgemisch (Kryopräzipitat) | $2 \times 10^5$ |
| 1. nach Tensid-Behandlung | $10 \times 10^4$ |
| 2. nach Adsorptionsbehandlung mit kolloidaler Kieselsäure | $2 \times 10^5$ |
| 3. nach ß-Propiolacton-Behandlung | $2 \times 10^3$ |
| 4. nach UV-Bestrahlung | $\emptyset$ |

Die Ankonzentrierung der Viren von Schritt 1. nach 2. erklärt sich durch einen Faktor-VIII-Ankonzentrierungsschritt, der mit einer Volumenreduktion um eine Zehnerpotenz verbunden ist.

### Präparat

### Herstellung einer Faktor-VIII-haltigen Lösung

9 Teile Spender-Venenblut wurden zu 1 Teil einer 3,8%igen Natriumcitrat-Stabilisatorlösung gegeben. Das Blut wurde möglichst bald nach der Blutentnahme zentrifugiert und die in physiologischer Kochsalzlösung aufgeschlemmten Erythrozyten dem Spender reinfundiert. Das Plasma wurde spätestens innerhalb von 48 Stunden bei -40°C eingefroren.

Aus dem gefrorenen Plasma wurde in bekannter Weise durch Auftauen auf +4°C und anschließende Zentrifugation das Kryopräzipitat vom Restplasma abgetrennt.

Die Erfindung wird durch die nachstehenden Beispiele näher erläutert.

Beispiel 1

Kaltsterilisation einer Faktor-VIII-haltigen Lösung in der Stufenfolge A-B-C-D

A. Behandlung mit physiologisch verträglichem, nichtionogenem Tensid

Eine gemäß Präparat erhaltene, in Form von Kryopräzipitat vorliegende Faktor-VIII-haltige Lösung wurde bei +37°C aufgetaut und bei Raumtemperatur mit einer solchen Menge an 0,02 M Tris-Puffer pH 6,5 (Tris(hydroxymethyl)-aminomethan) versetzt, daß pro 1 l Ausgangsplasma 100 ml Tris-Puffer angewendet wurden. Das Gemisch wurde mit Polyoxyethylen-20-sorbitan-monooleat (TWEEN$^R$ 80 der Fa. Atlas Chemie, Essen) in einer Konzentration von 3 Gew.-% versetzt und sodann 1 Std. bei Raumtemperatur gerührt und anschließend zentrifugiert. Der Niederschlag wurde verworfen.

B. UV-Bestrahlung

Der in Stufe A erhaltene Überstand wurde mit Hilfe eines UV-Durchfluß-Bestrahlungsgerätes bei 254 µm mit 2 mWatt/cm² x Min. UV-Licht bestrahlt. Die UV-bestrahlte Lösung wurde auf +4°C abgekühlt und anschließend mit Polyethylenglykol (mit einem mittleren Molekulargewicht von 4 000) bis zu einer PEG-Konzentration von 1 % versetzt, worauf das Gemisch 15 Min. gerührt und anschließend zentrifugiert wurde. Der Niederschlag wurde verworfen. Der Überstand wurde durch Zugabe von Polyethylenglykol 4000 auf eine PEG-Konzentration von 10 % gebracht. Der dabei entstandene Faktor-VIII-Niederschlag wurde durch Zentrifugieren abgetrennt und in einer Pufferlösung gelöst.

C. Behandlung mit ß-Propiolacton

Die in Stufe B erhaltene Faktor-VIII-Lösung wurde mit 0,05 % (Vol./Vol.) ß-Propiolacton versetzt und 1 Std. bei Raumtemperatur bei pH 6,5 gehalten.

D. Adsorptionsbehandlung mit kolloidaler Kieselsäure

Die in Stufe C erhaltene, mit ß-Propiolacton behandelte Faktor-VIII-Lösung wurde mit 3 Gew.-% an kolloidaler Kieselsäure mit einer Teilchengröße von 8 nm und einer spezifischen Oberfläche von 380 m²/g ("Aerosil 380" der Fa. Degussa) versetzt und 2 Stunden bei Raumtemperatur gerührt. Anschließend wurde zentrifugiert. Der Niederschlag wurde verworfen.

Die so erhaltene Faktor-VIII-Lösung wurde durch sterile Membranfilterscheiben steril filtriert, abgefüllt und gefriergetrocknet.

Beispiel 2

Kaltsterilisation einer Faktor-VIII-haltigen Lösung in der Stufenfolge A-D-C-B.

A. Behandlung mit physiologisch verträglichem, nichtionogenem Tensid.

Diese Behandlung wurde nach der Arbeitsweise von Beispiel 1 A durchgeführt.

D. Adsorptionsbehandlung mit kolloidaler Kieselsäure

Die in Stufe A erhaltene Faktor-VIII-Lösung wurde 2 Stunden bei 37°C mit 3 Gew.-% kolloidaler Kieselsäure ("Aerosil 380") gerührt und anschließend zentrifugiert. Der Niederschlag wurde verworfen.

C. Behandlung mit ß-Propiolacton

Die in Stufe D erhaltene Faktor-VIII-Lösung wurde bis zu einer Konzentration von 0,05 % (Vol./Vol.) mit ß-Propiolacton versetzt und 1 Std. bei Raumtemperatur bei pH 6,5 gehalten.

**B. UV-Bestrahlung**

Die in Stufe C erhaltene, mit ß-Propiolacton behandelte Lösung wurde unter Anwendung eines UV-Durchfluß-Bestrahlungsgerätes bei 254 µm mit 2 mWatt/cm² x Min. UV-Licht bestrahlt.

Die UV-bestrahlte Faktor-VIII-Lösung wurde durch sterile Membranfilterscheiben steril filtriert, abgefüllt und gefriergetrocknet.

Patentansprüche:

1. Verfahren zur Kaltsterilisation von Blutgerinnungsfaktor VIII enthaltenden Präparaten unter Behandlung mit ß-Propiolacton und Bestrahlung mit UV-Licht, dadurch gekennzeichnet, daß man eine Faktor VIII enthaltende Proteinlösung

A  mit einem physiologisch verträglichen, nichtionogenen Tensid behandelt;

B  einer Bestrahlung mit UV-Licht unterwirft;

C  mit ß-Propiolacton behandelt und

D  einer Adsorptionsbehandlung mit kolloidaler Kieselsäure unterwirft,

wobei die Stufen B-C-D auch in der Reihenfolge B-D-C oder D-C-B oder D-B-C durchgeführt werden können.

2.  Verfahren nach Anspruch 1, dadurch gekennzeichnet, daß man bei Anwendung der Stufenfolge B-C-D die mit UV-Licht bestrahlte Lösung fraktioniert, bevor sie mit ß-Propiolacton behandelt wird.

3.  Verfahren nach Anspruch 1 oder 2, dadurch gekennzeichnet, daß man als physiologisch verträgliches, nicht ionogenes Tensid Sorbitan-fettsäureester oder Poly-(deren) oxyethylenderivate in einer Konzentration von 1,0 bis 5 % verwendet.

4.  Verfahren nach einem der Ansprüche 1 bis 3, dadurch gekennzeichnet, daß man mit UV-Licht einer Intensität von 2 mWatt/cm² x Min. bestrahlt.

5. Verfahren nach einem der Ansprüche 1 bis 4, dadurch gekennzeichnet, daß man ß-Propiolacton in einer Konzentration von 0,012 bis 0,067 g pro g Protein verwendet.

6. Verfahren nach einem der Ansprüche 1 bis 5, dadurch gekennzeichnet, daß man kolloidale Kieselsäure mit einer spezifischen Oberfläche von 50 bis 400 m²/g in einer Konzentration von 1 bis 3 g pro g Protein verwendet.

Fig. 1

**Adsorptionsbehandlung von Plasma und einer Faktor VIII-Lösung mit kolloidaler Kieselsäure Adsorption**

Plasma
- △ Protein
- □ Fibrinogen

Faktor VIII-Lösung
- △ Protein
- □ Fibrinogen

Protein- und Fibrinogen Konzentration in % vom Ausgang

100 90 80 70 60 50 40 30 20 10

Ausgang
1. Aerosil 3 %, R.T., 2 h
2. Aerosil 3 %, R.T., 2 h
Ausgang
1. Aerosil 3 %, R.T., 2 h
2. Aerosil 3 %, R.T., 2 h

**0047462**

- 2/3 -

**Fig. 2** — Adsorptionsbehandlung von Plasma und einer Faktor VIII-Lösung mit koloidaler Kieselsäure

Fig. 3

Abhängigkeit der Faktor VIII-Aktivität von
der ß-Propiolactonkonzentration gereinigter F. VIII Lösungen

Faktor VIII-Aktivität (% d. N.)

3000

2000

1000

0.05          0.10          0.15          ——→ ß-PL(Vol. %)

- 3/3 -

0047462